(19) Europäisches Patentamt
European Patent Office
Office européen des brevets

(11) Veröffentlichungsnummer: 0 349 941
A2

(12) # EUROPÄISCHE PATENTANMELDUNG

(21) Anmeldenummer: 89112063.6

(22) Anmeldetag: 01.07.89

(51) Int. Cl.4: C07H 15/252 , C07H 19/24 , A61K 31/70

(30) Priorität: 08.07.88 DE 3823224

(43) Veröffentlichungstag der Anmeldung: 10.01.90 Patentblatt 90/02

(84) Benannte Vertragsstaaten: AT BE CH DE ES FR GB GR IT LI LU NL SE

(71) Anmelder: BEHRINGWERKE Aktiengesellschaft
Postfach 1140
D-3550 Marburg 1(DE)

(72) Erfinder: Kolar, Cenek, Dr.
Deutschhausstrasse 20
D-3550 Marburg(DE)
Erfinder: Kraemer, Hans Peter, Dr. Dr.
Birkenweg 16
D-3550 Marburg(DE)
Erfinder: Gerken, Manfred, Dr.
Wannkopfstrasse 12
D-3550 Marburg(DE)
Erfinder: Knödler, Ursula
Freiherr-vom Stein-Strasse 2
D-3557 Ebsdorfergrund 8(DE)
Erfinder: Hermentin, Peter, Dr.
Barfüssertor 30
D-3550 Marburg(DE)

(74) Vertreter: Klein, Otto, Dr. et al
Hoechst AG Zentrale Patentabteilung
Postfach 80 03 20
D-6230 Frankfurt am Main 80(DE)

(54) **Morpholino-Derivate der Rhodomycine, Verfahren zu ihrer Herstellung und ihre Verwendung.**

(57) Es werden neue zytostatisch wirksame Anthracyclin-Derivate, speziell 7-0-Glycosyl-ß und epsilon-(iso)-rhodomycinon-Derivate, die im Kohlenhydratteil des Moleküls eine modifizierte Morpholino-Struktur enthalten, und der Formel I

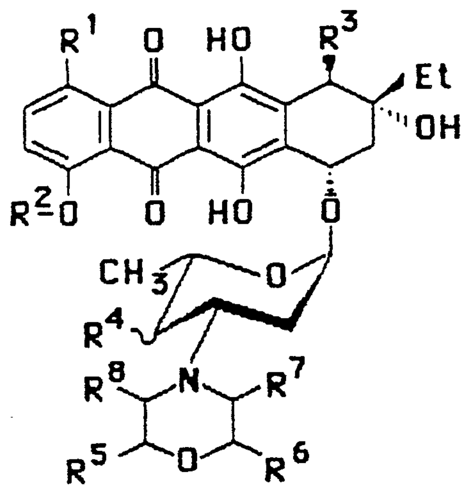

Formel I

entsprechen, beschrieben, in der die Reste folgende Bedeutung haben:
$R^1$ ist ein Wasserstoffatom oder eine Hydroxygruppe,

EP 0 349 941 A2

$R^2$ ist ein Wasserstoffatom oder eine Methylgruppe,
$R^3$ ist eine Methyloxycarbonyl- oder eine Hydroxygruppe,
$R^4$ ist ein Wasserstoffatom oder eine Hydroxygruppe,
$R^5$ ist ein Wasserstoffatom, eine Methylgruppe oder eine Hydroxymethylgruppe,
$R^6$ ist eine $C_{1-4}$-Alkyloxygruppe, Azidogruppe oder Cyanogruppe oder ein 9-N-Adenin-, 9-N-Guanin-, 1-N-Cytosin, 1-N-Uracil- oder 1-N-Thymin-Rest,
$R^6$ kann auch ein Wasserstoffatom sein, wenn $R^1$ = OH und $R^3$ = $COOCH_3$ sind,
$R^5$ und $R^6$ sind zusammen $-O-CH_2-$ und
$R^7$ oder $R^8$ sind ein Wasserstoffatom oder eine Cyanogruppe
sowie ein Verfahren zu ihrer Herstellung, sowie ihre Verwendung als Arzneimittel.

# Morpholino-Derivate der Rhodomycine, Verfahren zu ihrer Herstellung und ihre Verwendung

Die vorliegende Erfindung bezieht sich auf neue zytostatisch wirksame Anthracyclin-Derivate, und sie betrifft speziell 7-0-Glycosyl-ß und epsilon-(iso)Rhodomycinon-Derivate, die im Kohlenhydratteil des Moleküls eine modifizierte Morpholino-Struktur enthalten, Verfahren zu ihrer Herstellung sowie ihre Verwendung als Arzneimittel.

Die Substanzklasse der Anthracycline ist in der Fachliteratur eingehend beschrieben. Doxorubicin und sein 14-Desoxyanalogon, Daunorubicin, sind hier als die erfolgreichsten Vertreter dieser Substanzklasse genannt, die in der Klinik zur Behandlung einer großen Anzahl von festen Tumoren und Leukämien eingesetzt werden. Der Erfolg dieser speziellen Verbindungen ist jedoch typischerweise nicht bei allen Patienten gleich, und die Erfolgsrate ist bei einigen speziellen Tumorarten wie Dickdarmkrebs und Melanom geringer. Nebenwirkungen der Behandlung mit Doxorubicin und Daunorubicin sind u. a. eine Schädigung des Kreislaufsystems und dafür charakteristische Beschwerden.

Eine Anzahl weiterer Analoga, die sowohl in dem Aglyconteil als auch in dem Kohlenhydratteil modifiziert wurden, ist ferner beschrieben. Die relevante bis dato bekannte Literatur ist im folgenden angegeben. Acton et al. beschreiben in Anthracycline Antibiotics Editor H. S. El Khadem, 119 - 139, Academic Press (1982) u. a. Morpholi no-Derivate der Anthracycline, ihre Herstellung und biologische Resultate. Weitere Morpholino-Derivate, die sich vor allem von den Stammverbindungen Doxorubicin und Daunorubicin ableiten, wurden in DE 33 25 816 A1 beschrieben.

In der Stoffgruppe der ß-Rhodomycine wurde bisher ein Morpholino-Derivat von 7-0-(alpha-L-Daunosaminyl)-ß-rhodomycinon von H. Umezawa et al. (J. Antibiotics 40/7, 1058 - 1061 (1987)) beschrieben.

Morpholino-Derivate der Stoffgruppe epsilon-(iso)Rhodomycine wurden bisher nicht beschrieben.

Die bisher untersuchten Morpholino-, vor allem die Cyanomorpholino-Derivate der Anthracycline sind meist erheblich zytotoxischer als die Stammverbindungen Doxorubicin und Daunorubicin.

Aufgrund der hohen Toxizität dieser Verbindungen ist ihre therapeutische Anwendung enger und ihre Handhabung problematisch.

Ausgehend von diesem Stand der Technik ist es die Aufgabe der vorliegenden Erfindung, neue Anthracycline zu schaffen, die sich durch ein neues Wirkungsspektrum und eine geringere Toxizität auszeichnen.

Überraschenderweise hat es sich bei Untersuchungen von Morpholino-Derivaten der epsilon-(iso)-Rhodomycine gezeigt, daß 7-0-(3′-Morpholino-3′-desamino-alpha-L-daunosaminyl)-epsilon-isorhodomycinon (Abb. 1) in dem L1210 Tumormodell in vivo nur eine marginale Wirkung aufweist, aber dagegen die strukturverwandte Verbindung (Abb. 2), die ausgehend vom Oxidationsprodukt des alpha-Methylgalactopyranosides und 7-0-(alpha-L-Daunosaminyl)-epsilon isorhodomycinon hergestellt wurde, in diesem Modell kurativ wirksam ist. Hiermit eröffnete sich ein Weg zur Herstellung und Anwendung von neuen Rhodomycin-Derivaten.

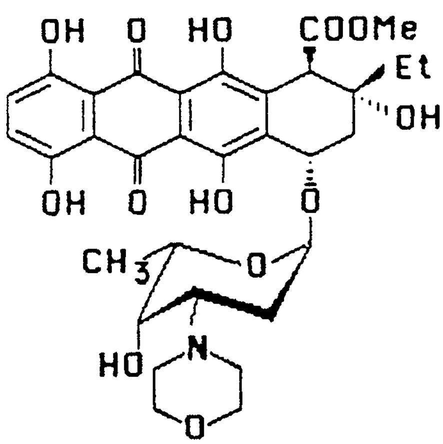

Abb. 1

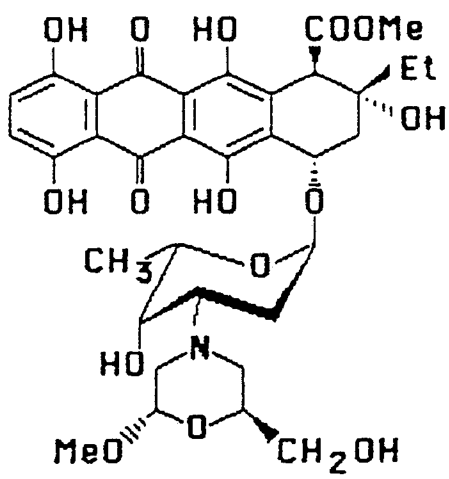

Abb. 2

Aufbauend auf diesen Erkenntnissen, hat es sich die vorliegende Erfindung zur Aufgabe gestellt, ausgehend von Oxidationsprodukten der Kohlenhydrate und semisynthetischen ß- oder epsilon-(iso)-

Rhodomycin-Derivaten, die einen 3-Aminozucker enthalten, neue modifizierte Morpholino-Derivate herzustellen, deren Eigenschaften eine Anwendung als tumor-therapeutisches Mittel erlaubt.

Gelöst wird diese erfindungsgemäße Aufgabe mit neuen zytostatisch wirksamen Anthracyclin-Derivaten, die der nachfolgenden allgemeinen Formel I

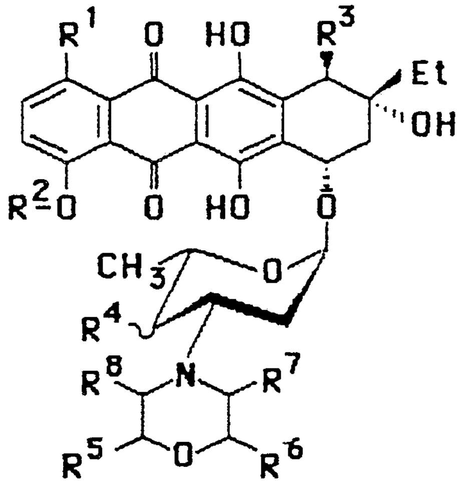

I

entsprechen, in der die Reste folgende Bedeutung haben:

$R^1$ ist ein Wasserstoffatom oder eine Hydroxygruppe,
$R^2$ ist ein Wasserstoffatom oder eine Methylgruppe,
$R^3$ ist eine Methyloxycarbonyl- oder eine Hydroxygruppe,
$R^4$ ist ein Wasserstoffatom oder eine Hydroxygruppe,
$R^5$ ist ein Wasserstoffatom, eine Methylgruppe oder eine Hydroxymethylgruppe,
$R^6$ ist eine $C_{1-4}$-Alkyloxygruppe, Azidogruppe oder Cyano-gruppe oder ein 9-N-Adenin-, 9-N-Guanin-, 1-N-Cytosin-, 1-N-Uracil- oder 1-N-Thymin-Rest,
$R^6$ kann auch ein Wasserstoffatom sein, wenn $R^1$ = OH und
$R^3$ = $COOCH_3$ sind,
$R^5$ und $R^6$ sind zusammen $-O-CH_2-$ und
$R^7$ und $R^8$ sind ein Wasserstoffatom oder eine Cyano-gruppe.

Bevorzugt sind Verbindungen der Formel I, worin
$R^6$ = $OCH_3$, CN, $N_3$ oder 9-N-Adenin oder
$R^5$ und $R^6$ zusammen $-0-CH_2-$ sind und die übrigen Reste die gerade angegebene Bedeutung haben.

Das erfindungsgemäße Verfahren zur Herstellung einer der neuen Verbindungen der Formel I ist dadurch gekennzeichnet, daß man

a) in an sich bekannter Weise ein 0- oder N-Glycopyranosid oder -furanosid eines Kohlenhydrat-Derivats der Formel II durch Perjodsäure-Oxidation in ein Dialdehyd-Derivat der Formel III überführt,

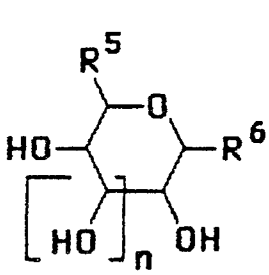

Formel II

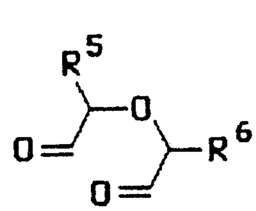

Formel III

wobei die Reste in den Formeln II und III folgende Bedeutung haben:

$R^5$ ist ein Wasserstoffatom, eine Methylgruppe oder eine Hydroxymethylgruppe,
$R^6$ ist ein Wasserstoffatom, eine $C_{1-4}$-Alkyloxygruppe, Azidogruppe, Cyanogruppe, 9-N-Adenin-, 9-N-Guanin-, 1-N-Cytosin-, 1-N-Uracil oder 1-N-Thymin-Rest oder
$R^5$ und $R^6$ sind zusammen $-O-CH_2-$ und n = 0 oder 1,

b) ein Rhodomycinglycosid der Formel IV,

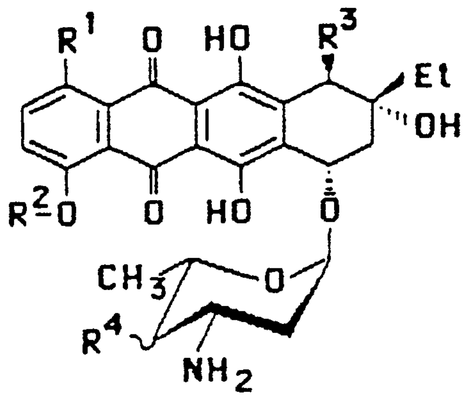

Formel IV

worin
$R^1$ ein Wasserstoffatom oder eine Hydroxygruppe,
$R^2$ ein Wasserstoffatom oder eine Methylgruppe,
$R^3$ eine Methyloxycarbonyl- oder eine Hydroxygruppe und
$R^4$ ein Wasserstoffatom oder eine Hydroxygruppe sind,
oder eines seiner Additionssalze mit einem Überschuß eines Dialdehydes der Formel III in Anwesenheit von 1 bis 6 Äquivalenten eines Alkalimetallcyanoborhydrides und eines polaren organischen Lösungsmittels wie Methanol, Acetonitril oder dessen wäßrigen Gemischen bei 10° C bis 50° C umsetzt zu einem Produktoemisch, bestehend aus einem Morpholino-Derivat der Formel I, worin die Reste $R^1$, $R^2$, $R^3$, $R^4$, $R^5$ und $R^6$ ihre vorher genannte Bedeutung beibehalten und $R^7$ und $R^8$ Wasserstoffatome sind, und einem Cyano-Morpholino-Derivat der Formel I,
worin die Reste $R^1$, $R^2$, $R^3$, $R^4$, $R^5$ und $R^6$ ihre vorher genannte Bedeutung beibehalten und $R^7$ oder $R^8$ ein Wasserstoffatom oder eine Cyanogruppe ist, wobei die beiden Derivate isoliert werden, vorzugsweise säulenchromatographisch an Kieselgel unter Verwendung eines Eluierungssystems aus Methylenchlorid/Aceton oder Methanol (95 -80/5 Vol./Vol.) und eines Zusatzes von Essigsäure und Triethylamin.

Gegenstand der Erfindung sind auch Arzneimittel, die eine oder mehrere der Verbindungen der Formel I als Wirkstoff enthalten.

Neben den üblichen pharmazeutischen Konfektionierungs-und/oder Verdünnungsmitteln können diese Arzneimittel neben den Verbindungen der Formel I zur Unterstützung der Therapie gegebenenfalls auch noch weitere Wirkstoffe enthalten, sofern diese mit den erfindungsgemäßen Verbindungen der Formel I zusammen keine unerwünschten Nebenwirkungen zeigen. Die Dosierungs- und Anwendungsweise entspricht im wesentlichen der für Doxorubicin und Daunorubicin bekannten, wobei aufgrund der geringeren Toxizität der erfindungsgemäßen Verbindungen auch höhere Dosierungen und/oder eine häufigere Verabreichung in Frage kommen.

Die Bestimmung der zytostatischen Wirksamkeit der hier beschriebenen Verbindungen erfolgte in vitro an L1210-Leukämiezellen der Maus sowie A 549- und HT-29-Zellen oder in vivo an L1210-Leukämie, B16-Melanoma und Lewis Lung Adenocarcinoma. Die akute Toxizität der Verbindungen wurde an BDF1-Mäusen ermittelt. Die Methoden sowie Ergebnisse dieser Untersuchungen sind in dem experimentellen Teil beschrieben.

## Beispiele

In den folgenden Beispielen wird die vorliegende Erfindung näher beschrieben, ohne sie hierbei einzuschränken.

Beispiel 1

Herstellung von Dialdehyden, ausgehend von Kohlenhydrat-Derivaten

1′(R)-Hydroxymethyl-1(S)-methoxy-2,2′-oxydiacetaldehyd (Verbindung 1)

1 g (5.1 mmol) Methyl alpha-D-galactopyranosid in 20 ml Ethanol wurde portionsweise mit 100 ml 0.1 M wäßriger Natriumperjodat-Lösung bei Raumtemperatur versetzt. Nach 3 Stunden Rühren wurde der Reaktionsansatz in Vakuum eingedampft. Der Rückstand wurde in Methanol suspendiert und die anorganischen Salze wurden abfiltriert. Nach Eindampfen der methanolischen Lösung wurde der resultierende Sirup in Essigsäureethylester gelöst, mit Natriumsulfat verrührt, filtriert und die Lösung im Vakuum eingedampft und der Rückstand im Hochvakuum getrocknet. Das dünnschichtchromatographisch einheitliche Produkt (Laufmittel: Chloroform/Methanol 5:1 oder Chloroform/Methanol/Eisessig/Wasser 8:4:2:1) wurde für die nächste Stufe ohne weitere Reinigung verwendet.
Ausbeute: 830 mg (Rohprodukt), Rf = 0.76 in Chloroform/Methanol 5:1

1′(R)-Hydroxymethyl-1(R)-methoxy-2,2′-oxydiacetaldehyd (Verbindung 2)

Ausgehend von 1.0 g Methyl ß-D-galactopyranosid wurde die Titelverbindung nach der obigen Vorschrift hergestellt. Ausbeute: 835 mg (Rohprodukt), Rf. = 0.78 in Chloroform/Methanol 5:1

1(S)-Methoxy-1,(S)-methyl-2,2′-oxydiacetaldehyd (Verbindung 3)

Ausgehend von 1 g (5.6 mmol) Methyl ß-L-fucopyranosid wurde die Titelverbindung nach der Vorschrift zur Herstellung von Verbindung 1 hergestellt.
Ausbeute: 820 mg (Rohprodukt), Rf = 0.8 in Chloroform/Methanol 5:1

1(S)-Cyano-1′(R)-hydroxymethyl-2,2′-oxydiacetaldehyd (Verbindung 4)

Ausgehend von 1 g (5.61 mmol) 1-Cyano-1-desoxy-alpha-D-mannopyranose und 120 ml 0.1 M Natriumperjodat-Lösung wurde die Titelverbindung nach der Vorschrift zur Herstellung von Verbindung 1 dargestellt.
Ausbeute: 830 mg (Rohprodukt), Rf = 0.58 in Chloroform/Methanol 5:1

1(S)-Azido-1′(R)-hydroxymethyl-2,2′-oxydiacetaldehyd (Verbindung 5)

Ausgehend von 1 g (4.87 mmol) alpha-L-Mannopyranosylazid wurde die Titelverbindung nach der Vorschrift zur Herstellung von Verbindung 1 dargestellt.
Ausbeute: 850 mg (Rohprodukt), Rf = 0.60 in Chloroform/Methanol 5:1

1,3-Dioxolan-2(R),2′(R)-dicarbaldehyd (Verbindung 6)

Ausgehend von 1 g (6.17 mmol) 1,6-Anhydro-ß-D-mannopyranose wurde die Titelverbindung nach der Vorschrift zur Herstellung von Verbindung 1 dargestellt.
Ausbeute: 800 mg (Rohprodukt), Rf = 0.55 in Chloroform/Methanol 5:1

1(S)-Adenin-9-yl-1,(R)-hydroxymethyl-2,2′-oxydiacetaldehyd (Verbindung 7)

Ausgehend von 1 g (3.74 mmol) Adenosin und 70 ml 0.1 M Natriumperjodat-Lösung wurde die Titelverbindung nach der Vorschrift zur Herstellung von Verbindung 1 hergestellt.

Ausbeute: 990 mg (Rohprodukt), Rf = 0.67 in Chloroform/Methanol 2:1

Beispiel 2

Herstellung von Morpholino-Derivaten der epsilon-Isorhodomycine

7-0-(3′-(6″(s)-Hydroxymethyl-2″(s)-methoxy-4″-morpholinyl)-2′,3′,6′-tri-desoxy-alpha-L-lyxohexopyranosyl)-epsilon-isorhodomycinon (Verbindung 8)

270 mg (0.47 mmol) 7-0-(alpha-L-Daunosaminyl)-epsilonisorhodomycinon wurden in 10 ml Methanol gelöst und mit 29.6 mg (0.47 mmol) Natriumcyanoborhydrid und 152.7 mg (0.942 mmol) Dialdehyd-Verbindung 1, gelöst in 1.5 ml Methanol, versetzt. Nach 30 min Rühren bei Raumtemperatur wurden weitere 152.7 mg (2 äg.) Dialdehyd-Verbindung 1 zugegeben. Der Reaktionsansatz wurde nach 2 h eingedampft, und der Rückstand wurde chromatographisch gereinigt (Kieselgel, Laufmittel: Chloroform/Aceton/Eisessig/Wasser/Triethylamin 95:5:1:0.25:0.1).
Ausbeute: 202 mg (62 %) $(alpha)_D = +686^{\circ}$ (c = 0.1 in Chloroform), MS-FAB $(M+H^+)$ m/e = 704, Schmelzpunkt = 173-175° C

Die folgenden Verbindungen 9 bis 14 wurden nach der Vorschrift zur Herstellung der Verbindung 8 dargestellt.

7-0-(3′-(6″(S)-Hydroxymethyl-2″(R)-methoxy-4″-morpholinyl)-2′,3′,6′-tri-desoxy-alpha-L-lyxohexopyranosyl)-epsilon-isorhodomycinon (Verbindung 9)

Ausgangsverbindungen: 270 mg 7-0-(alpha-L-Daunosaminyl)epsilon-isorhodomycinon, 29.6 mg Natriumcyanoborhydrid und 305 mg Verbindung 2
Ausbeute: 200 mg (61 %), MS-FAB $(M+H^+)$ m/e = 704

7-0-(3′-(6″(S)-methyl-2″(S)-methoxy-4″-morpholinyl)-2′, 3′,6′-tridesoxy-alpha-L-lyxohexopyranosyl)-epsilonisorhodomycinon (Verbindung 10)

Ausgangsverbindungen: 250 mg (0.436 mmol) 7-0-(alpha-L-Daunosaminyl)-epsilon-isorhodomycinon, 27.4 mg Natriumcyanoborhydrid und 127.4 mg Verbindung 3
Ausbeute: 191.8 mg (64 %)

7-0-(3′-(6″(S)-Hydroxymethyl-2″(S)-cyano-4″-morpholinyl)-2′,3′,6′-tri-desoxy-alpha-L-lyxohexopyranosyl)-epsilonisorhodomycinon (Verbindung 11)

Ausgangsverbindungen: 250 mg (0.436 mmol) 7-0-(alpha-L-Daunosaminyl)-epsilon-isorhodomycinon, 27.4 mg Natriumcyanoborhydrid und 151 mg Verbindung 4
Ausbeute: 173 mg (57 %)

7-0-(3′-(6″(S)-Hydroxymethyl-2″(S)-azido-4″-morpholinyl)-2′,3′,6′-tri-desoxy-alpha-L-lyxohexopyranosyl)-epsilonisorhodomycinon (Verbindung 12)

Ausgangsverbindungen: 250 mg (0.436 mmol) 7-0-(alpha-L-Daunosaminyl)-epsilon-isorhodomycinon, 27.4 mg Natriumcyanoborhydrid und 151 mg (2 äq.) Verbindung 5
Ausbeute: 174 mg (56 %)

7-0-(3′-(2″,6″-Epoximethano-4″-morpholinyl)-2′,3′,6′-tridesoxy-alpha-L-lyxohexopyranosyl)-epsilon-isorhodomycinon (Verbindung 13)

Ausgangsverbindungen: 250 mg (0.436 mmol) 7-0-(alpha-L-Daunosaminyl)-epsilon-isorhodomycinon, 27.4 mg Natriumcyanoborhydrid und 113 mg (2 äq.) Verbindung 6
Ausbeute: 181 mg (62 %) MS-FAB ($M+H^+$) m/e = 672

7-0-(3′-(2″(R)-(9‴-Adeninyl)-6″(S)-hydroxymethyl)-4″-morpholinyl)-2′,3′,6′-tri-desoxy-alpha-L-lyxohexopyranosyl)-epsilon-isorhodomycinon (Verbindung 14)

Ausgangsverbindungen: 250 mg (0.436 mmol) 7-0-(alpha-L-Daunosaminyl)-epsilon-isorhodomycinon, 27.4 mg Natriumcyanoborhydrid und 231 mg Verbindung 7
Ausbeute: 253 mg (72 %) MS-FAB ($M+H^+$) m/e = 807

7-0-(3′-(6″(S)-Hydroxymethyl-2″(S)-methoxy-4″-morpholinyl)-2′,3′,6′-tri-desoxy-alpha-L-arabinohexopyranosyl)epsilon-isorhodomycinon (Verbindung 15)

Ausgangsverbindungen: 270 mg (0.47 mmol) 7-0-(alpha-L-Acosaminyl)-epsilon-isorhodomycinon, 29.6 mg Natriumcyanoborhydrid und 152.7 mg Verbindung 1
Ausbeute: 200 mg (61 %)
$^1$H-NMR (300 MHz, H,H-COSY, $CDCl_3$, delta (ppm)) = 7.26 s (H-2 und H-3), 5.19 bs (H-7 J = 2.5 Hz), 2.35 d (H-8a, J = 15 Hz), 2.19 dd (H-8b J = 15 Hz und J = 2.5 Hz), 4.25 s (H-10), 1.82 m (H-13a), 1.41 m (H-13b), 1.11 t (H-14 J = 7.2), 12.94 s und 12.29 s (PhOH), 3.69 s (COOMe), 4.30 bs (9-OH), 5.51 d (H-1′) 1.66 ddd (H-2a, J = 13 Hz, J = 13 Hz, J = 4 Hz), 1.89 dd (H-2′e, J = 13 Hz, J = 3 Hz), 2.61 m (H-3′), 3.22 t (H-4′, J = 10 Hz, J = 9 Hz), 3.89 m (H-5′), 1.35 d (H-6′, J = 6.5 Hz), 4.58 s (H-2″), 2.65 d (H-3″a), 2.52 d (H-3″e, J = 11.5 Hz), 2.22 t (H-5″a), 2.64 d (H-5″e), 3.95 m (H-6″), 3.64 dd ($CH_2OH$″a, J = 12 Hz, J = 4 Hz), 3.54 dd ($CH_2OH$″b, J = 12 Hz, J = 5.5 Hz), 3.31 s (MeO)

Beispiel 3

Herstellung von Morpholino-Derivaten der ß-Rhodomycine

7-0-(3′-(6″(S)-Hydroxymethyl-2″(S)-methoxy-4″-morpholinyl)-2′,3′,6′-tri-desoxy-alpha-L-lyxohexopyranosyl)-ß-rhodomycinon (Verbindung 16)

300 mg (0.582 mmol) 7-0-(alpha-L-Daunosaminyl)-ß-rhodomycinon wurden in 10 ml Acetonitril gelöst und mit 36.6 mg Natriumcyanoborhydrid und 188.7 mg Verbindung 1, gelöst in 2 ml Methanol, bei -15° C versetzt. Die Reaktionsmischung wurde 2 h bei Raumtemperatur gerührt und anschließend in Vakuum eingeengt. Der Rückstand wurde chromatographisch gereinigt (Kieselgel, Laufmittel: Chloroform/Aceton/Eisessig/Wasser/Triethylamin 92:8:1:0.25:0.1).
Ausbeute: 244 mg (65 %), $(alpha)_D$ = +520° (c = 0.05 in Chloroform/ Methanol 1:1), Schmelzpunkt = 198-200° C
$^1$H-NMR (300 MHz, H,H-COSY, $CDCl_3$, delta (ppm)) =
7.62 d (H-1, J = 7.5 Hz), 7.48 t (H-2), 7.06 d (H-3, J = 8.5), 4.91 s (H-7), 1.93 m (H-8a), 1.93 m (H-8b), 4.58 s (H-10), 1.56 m (H-13a, H-13b), 0.84 t (H-14, J = 7.5), 5.26 s (H-1′), 1.63 m (H-2′a, H-2′b), 2.06 m (H-3), 3.46 s (H-4′), 3.82 m (H-5′), 1.09 d (H-6′, J = 6.5 Hz), 4.43 s (H-2″), 1.97 m (H-2″a), 2.79 d (H-2″b, J = 11.5), 1.63 m (H-5″a), 2.59 d (H-5″b, J = 10.5 Hz), 3.66 m (H-6″), 4.24 s ($CH_2OH$), 3.32 s (MeO)

Die folgenden Verbindungen 17 bis 20 wurden nach der Vorschrift zur Herstellung der Verbindung 16 dargestellt.

7-0-(3′-(6″(S)-Hydroxymethyl-2″(R)-methoxy-4″-morpholinyl)-2′,3′,6′-tri-desoxy-alpha-L-lyxohexopyranosyl)-ß-rhodomycinon (Verbindung 17)

Ausgangsverbindungen: 300 mg (0.582 mmol) 7-0-(alpha-L-Daunosaminyl)-ß-rhodomycinon, 36.6 mg (1 äq.) Natriumcyanoborhydrid und 188.7 mg (2 äq.) Verbindung 2

Ausbeute: 240 mg (64 %), MS-FAB ($M+H^+$) m/e = 646, ($M+Na^+$) m/e = 668

7-0-(3′-(6″(S)-Hydroxymethyl-2″(S)-cyano-4″-morpholinyl)-2′,3′,6,-tri-desoxy-alpha-L-lyxohexopyranosyl)-ß-rhodomycinon (Verbindung 18)

Ausgangsverbindungen: 300 mg (0.582 mmol) 7-0-(alpha-L-Daunosaminyl)-ß-rhodomycinon, 36.6 mg (1 äq.) Natriumcyanoborhydrid, 183 mg (2 äq.) Verbindung 4
Ausbeute: 201 mg (54 %)

7-0-(3′-(6″(S)-methyl-2″(S)-methoxy-4″-morpholinyl)-2′, 3′,6′-tridesoxy-alpha-L-lyxohexopyranosyl)-ß-rhodomycinon (Verbindung 19)

Ausgangsverbindungen: 300 mg (0.582 mmol) 7-0-(alpha-L-Daunosaminyl)-ß-rhodomycinon, 36.6 mg (1 äq.) Natriumcyanoborhydrid und 170 mg (2 äq.) Verbindung 3
Ausbeute: 230 mg (63 %)

7-0-(3′-(2″,6″-Epoximethano-4″-morpholinyl)-2′,3′,6′-tridesoxy-alpha-L-lyxohexopyranosyl)-ß-rhodomycinon (Verbindung 20)

Ausgangsverbindungen: 300 mg (0.582 mmol) 7-0-(alpha-L-Daunosaminyl)-ß-rhodomycinon, 36.6 mg (1 äq.)Natriumcyanoborhydrid, 151 mg (2 äq.) Verbindung 6
Ausbeute: 205 mg (58 %), MS-FAB ($M+H^+$) m/e = 614

## Beispiel 4

Herstellung von Morpholino-Derivaten der 4-0-Methyl-ß-rhodomycine

7-0-(3′-(6″(S)-Hydroxymethyl-2″(S)-methoxy-4″-morpholinyl)-2′,3′,6′-tri-desoxy-alpha-L-lyxohexopyranosyl)-4-0-methyl-ß-rhodomycinon (Verbindung 21)

250 mg (0.472 mmol) 7-0-(alpha-L-Daunosaminyl)-4-0-methyl-ß-rhodomycinon wurden in 10 ml Methanol gelöst und mit 29.7 mg (1 äq.) Natriumcyanoborhydrid und 153 mg (2 äq.) Verbindung 1 versetzt. Nach 2 h Rühren bei Raumtemperatur wurde der Reaktionsansatz in Vakuum eingedampft und der Rückstand wurde chromatographisch gereinigt (Kieselgel, Laufmittel: Chloroform/Aceton/Eisessig/Wasser/Triethylamin 95:5:2:0.25:0.1)
Ausbeute: 190 mg (62 %) MS-FAB ($M+H^+$) m/e = 650

7-0-(3′-2″(S)-Cyano-6″(S)-hydroxymethyl-4″-morpholinyl)-2′,3′,6′tri-desoxy-alpha-L-lyxohexopyranosyl)-4-0-methyl-ß-rhodomycinon (Verbindung 22)

Ausgehend von 250 mg (0.472 mmol)7-0-(alpha-L-Daunosaminyl)-4-0-methyl-ß-rhodomycinon, 29.7 mg (1 äq.) Natriumcyanoborhydrid und 149 mg (2 äq.) Verbindung 4 wurde die Titelverbindung nach der Vorschrift zur Herstellung von Verbindung 19 dargestellt.
Ausbeute: 207 mg (67 %) MS-FAB ($M+H^+$) m/e = 655

## Beispiel 5

Herstellung von 3″- oder 5″-Cyano-Morpholino-Derivaten

7-0-(3′-(3″ oder 5″-Cyano-6″(S)-hydroxymethyl-2″(S)-methoxy-4″-morpholinyl)-2′,3′,6′-tri-desoxy-alpha-L-lyxohexopyranosyl)-epsilon-isorhodomycinon (Verbindung 23)

270 mg (0.47 mmol) 7-0-(alpha-L-Daunosaminyl)-epsilonisorhodomycinon wurden in 10 ml Methanol gelöst und mit 29.6 mg (0.47 mmol) Natriumcyanoborhydrid, 30 mg Natrium cyanid und 152.7 mg (0.942 mmol) Dialdehydverbindung 1, gelöst in 2 ml Methanol/Wasser 1:1, versetzt. Nach 4 h Rühren bei Raumtemperatur wurde der Reaktionsansatz eingedampft, und der Rückstand wurde säulenchromatographisch gereinigt (Kieselgel, Laufmittel: Chloroform/Methanol/Essigsäure/Wasser/Triethylamin 4:2:1:0.6:0.02). Ausbeute: 106 mg (31 %), $(alpha)_D$ = +526° (c = 0.05 in Chloroform), Schmelzpunkt = 209-211° C, MS-FAB $(M+H^+)$ m/e = 729, (M-CN) m/e = 702

Die folgenden Verbindungen 24 und 25 wurden nach der Vorschrift zur Herstellung der Verbindung 23 dargestellt.

7-0-(3′-(3″-oder 5″-Cyano-6″(S)-hydroxymethyl-2″(S)-methoxy-4″-morpholinyl)-2′,3′,6′-tri-desoxy-alpha-L-arabinohexopyranosyl)-epsilon-isorhodomycinon (Verbindung 24)

Ausgangsverbindungen: 270 mg (0.47 mmol) 7-0-(alpha-L-Daunosaminyl)-epsilon-isorhodomycinon, 29.6 mg Natriumcyanoborhydrid, 30 mg Natriumcyanid und 152.7 mg Verbindung 1.
Ausbeute: 97 mg (28 %), $(alpha)_D$ = +356° (c = 0.05 in Chloroform), Schmelzpunkt = 220-222° C, MS-FAB $(M+H^+)$ m/e =729, (M-CN) m/e = 702

7-0-(3′-(3″- oder 5″-Cyano-6″(S)-hydroxymethyl-2″(S)methoxy-4″-morpholinyl)-2′,3′,6′-tri-desoxy-alpha-L-lyxohexopyranosyl)-ß-rhodomycinon (Verbindung 25)

Ausgangsverbindungen: 300 mg (0.582 mmol) 7-0-alpha-L-Daunosaminyl)-ß-rhodomycinon, 36.6 mg Natriumcyanoborhydrid, 40 mg Natriumcyanid und 188.7 mg Verbindung 1
Ausbeute: 117 mg (30 %) $(alpha)_D$ = +220° (c = 0.1 in Chloroform), Schmelzpunkt = 152-154° C, MS-FAB $(M+H^+)$ m/e = 671, (M-CN) m/e = 644

Beispiel 6

Herstellung von Morpholino- und 3″-Cyano-Morpholino-Derivaten

7-0-(3′-(4″-Morpholinyl)-2′,3′,6′-tridesoxy-alpha-L-lyxohexopyranosyl)-epsilon-isorhodomycinon (Verbindung 26)

und

7-0-(3′-(3″-Cyano-4″-morpholinyl)-2′,3′,6′-tridesoxyalpha-L-lyxohexopyranosyl)-epsilon-isorhodomycinon (Verbindung 27)

270 mg(0.47 mmol) 7-0-(alpha-L-Daunosaminyl)-epsilonisorhodomycinon wurden in 10 ml Methanol gelöst und mit 29.6 mg (0.47 mmol) Natriumcyanoborhydrid und 10 mg 2,2′-Oxy-bis-acetaldehyd versetzt. Der Reaktionsansatz wurde wie üblich aufgearbeitet. Die Verbindungen 26 und 27 wurden säulenchromatographisch an Kieselgel mit dem Laufmittel: Chloroform/Methanol/Essigsäure/Wasser/Triethylamin 4:2:1:0.6:0.02 getrennt und mittels NMR- und MS-Analyse charakterisiert.

4-0-Methyl-7-0-(3′-(4″-morpholinyl)-2′,3′,6′-tridesoxyalpha-L-lyxohexopyranosyl)-ß-rhodomycinon (Verbindung 28)

und

4-0-Methyl-7-0-(3′-(3″-cyano-4″-morpholinyl)-2′,3′,6′-tridesoxy-alpha-L-lyxohexopyranosyl)-ß-rhodomycinon (Verbindung 29)

Ausgehend von 7-0-(alpha-L-Daunosaminyl)-4-0-methyl-ß-rhodomycinon und 2,2′-Oxy-bisacetaldehyd wurden die Verbindungen 28 und 29 wie oben beschrieben hergestellt und mittels NMR- und MS-Analyse charakterisiert.

## Beispiel 7

Zytotoxizität gegen L1210 Leukämiezellen (stem cell assay)

Der Test wurde entsprechend der von Hamburger und Salmon beschriebenen Methodik mit den unten beschriebenen Modifikationen durchgeführt.

Konditioniertes Medium wurde durch McCoy 5A Medium ersetzt. Als Folge der hohen Klonierungsrate der L1210 Leukämiezellen in Soft Agar wurde die Anzahl an Tumorzellen pro Platte auf $5 \times 10^2$ reduziert.

Die Zellen wurden mit unterschiedlichen Konzentrationen der Testsubstanz für 1 h bei 37° C inkubiert. Anschließend wurden die Zellen zweimal mit McCoy 5A Medium gewaschen und anschließend in einem Zwei-Schichten-Agar System als obere Schicht entsprechend der Methode von Hamburger und Salmon ausplattiert.

Zusätzliche Parallelexperimente wurden unter Verwendung einer kontinuierlichen Inkubationszeit durchgeführt, wobei unterschiedliche Konzentrationen der Testsubstanz der oberen Agarschicht vor Ausplattieren der Zellen zugemischt wurden.

Die Platten wurden in einem Brutschrank mit 5 % $CO_2$, 20 % $O_2$ und 95 % relativer Luftfeuchte für 5-7 Tage inkubiert. Nach dieser Zeit wurden Kolonien mit einem Durchmesser von 60 µm mit Hilfe eines Invertoskopes gezählt.

Die Ergebnisse wurden angegeben als prozentualer Anteil der Kolonien in behandelten versus unbehandelten Gruppen. Der Variationskoeffizient bei wiederholten Experimenten war kleiner als 15 %.

Die an den getesteten Verbindungen erhaltenen Resultate sind in der Tabelle 1 enthalten.
Aus der Dosis-Wirkungskurve wurde die $IC_{50}$ für die kontinuierliche und einstündige Inkubation bestimmt (Tab. 1).

Die in der Tabelle zusammengefaßten Zahlen belegen, daß die meisten Substanzen eine sehr hohe Zytotoxizität ($IC_{50}$ 0.1 µg/ml) in unterschiedlichen in vitro Testsystemen gegenüber menschlichen und tierischen Tumorzellen aufweisen.

## Beispiel 8

Proliferationstest (MTT-Reduktion)

L1210, A 549 oder HT 29 in exponentieller Wachstumsphase wurden in einer Zelldichte von $5 \times 10^3$ Zellen/ml in RPMI 1640 in einer 96 Well Mikrotiterplatte für 72 h mit unterschiedlichen Konzentrationen der Testsubstanz bei 37° C, 5 % $CO_2$ und 95 % relativer Luftfeuchte inkubiert. Kontrollexperimente erhielten anstelle der Testsubstanz lediglich Wachstumsmedium. Für jede Testsubstanz sowie für die Kontrolle wurden 4fach Bestimmungen angesetzt. Nach 65 h Inkubation wurden 50 µl einer MTT-Lösung (2,5 mg/ml in Phosphat-gepuffertem Kochsalz) zugegeben. In Gegenwart lebender Zellen wurde MTT zu einem dunkelroten unlöslichen Formazanfarbstoff reduziert. Diese Reaktion war nach 7 h (L1210 Zellen) bzw. nach 24 h (A 549, HT 29 Zellen) beendet und das überstehende Medium wird sorgfältig abgesaugt. Der unlösliche Farbstoff wurde durch Zugabe von 100 µl DMSO aufgelöst und die Extinktion der so entstehenden Lösung anschließend für jedes Well in einem Multiscan Photometer 340 CC der Fa. Flow bei einer Wellenlänge von 492 nm vermessen.

Aus dem Verhältnis der Extinktionen behandelter und unbehandelter Zellen ergab sich eine Dosis-Wirkungskurve, aus der die Konzentration, die gerade 50 % der Zellen ($IC_{50}$) abtötet, abgelesen werden konnte. Für wiederholte Untersuchungen betrug der Variationskoeffizient weniger als 15 %.

Beispiel 9

Ermittlung der akuten Toxizität

Zur Ermittlung der akuten Toxizität wurden BDF1-Mäusen am Tag 0 unterschiedliche Dosen der Testsubstanz, gelöst in 0.5 ml 5 %iger Glucose-Lösung, intraperitoneal injiziert. Kontrollgruppen erhielten lediglich 0.5 ml 5 %ige Glucose-Lösung. Pro Konzentration der Testsubstanz wurden 5 Mäuse verwendet. Am Tag 14 wurde die Zahl der überlebenden Mäuse ermittelt und daraus nach der Lichtfield Wilcoxon Methode die LD5, LD50 und LD95 ermittelt. Die Toxizität (LD50 (mg/kg)) der hier beschriebenen Verbindungen im Vergleich zu Adriamycin wurde ermittelt.

Beispiel 10

In vivo Wirksamkeit der Rhodomycine gegen L1210 Leukämie der Maus

Ascitesflüssigkeit wurde unter sterilen Bedingungen DBA2 Mäusen (weiblich, 18 - 20 g) 7 Tage nach Implantation entnommen. Der Ascites wurde dreimal mit PBS gewaschen, gezählt und auf eine Zellzahl von $10^6$ in 0.2 ml PBS eingestellt. $10^6$ Zellen, suspendiert in 0.2 ml PBS, wurden anschließend DBF1 Mäusen (weiblich, 18 - 20 g) intraperitoneal injiziert. 6 Tiere pro Gruppe wurden für jede Substanzkonzentration bzw. als Kontrolle eingesetzt.

Zur Ermittlung der antitumoralen Wirksamkeit wurden die Tiere am Tag 1 und 5 nach Injektion der Testsubstanz gewogen. Gewichtsverlust von mehr als 20 % am Tag 5 wurde als Indikator einer toxischen Substanzwirkung angesehen.

Am Ende des Experimentes (Tod aller Tiere oder überlebende Tiere am Tag 60) wurde die mittlere Überlebenszeit der Tiere in den jeweiligen Gruppen bestimmt, sofern am Tag 5 des Experimentes mindestens 65 % der Tiere noch gelebt hatten. Die mittlere Überlebenszeit wurde ausschließlich für im Verlaufe des Experimentes sterbende Tiere bestimmt. Langzeitüberlebende (LTS) wurden bei dieser Berechnung nicht berücksichtigt und gesondert aufgeführt.

Aus der mittleren Überlebenszeit ($MST_T$) der behandelten Gruppen sowie der Kontrollgruppen ($MST_C$) wurde die antitumorale Wirksamkeit (T/C) für die jeweilige Substanzkonzentration in Prozent der unbehandelten Kontrolle entsprechend der folgenden Formel bestimmt:

$$T/C\ \% = \frac{MST_T}{MST_C} \times 100$$

T/C-Werte größer als 125 % wurden als Indikator einer signifikanten antitumoralen Wirksamkeit der Testsubstanz angesehen. Die Dosis, die den größten antitumoralen Effekt (optimale Dosierung) zeigte sowie jeweils eine Dosisstufe oberhalb und unterhalb dieser Dosis wurden ermittelt. Tiere, die am Tag 60 des Experimentes noch lebten, wurden als Longterm-Survivors getrennt aufgeführt.

Tabelle 1

| | IC₅₀ (µg/ml) | | | | |
|---|---|---|---|---|---|
| | MTT-ASSAY | | | STEM CELL ASSAY | |
| | | | | cont. exp. | 1 h exp. |
| Verbindung | L1210 | HT 29 | A 549 | L1210 | L1210 |
| VIII | 0.009 | 0.035 | 0.038 | 0.029 | 0.95 |
| IX | - | - | - | 0.12 | 1.0 |
| XIV | - | - | - | 1.0 | 0.5 |
| XV | 0.035 | 0.03 | 0.038 | 0.078 | 0.32 |
| XVI | 0.036 | 0.11 | 0.046 | 0.026 | 0.15 |
| XVII | - | - | - | 0.55 | 1.0 |
| XIX | - | - | - | 0.021 | 0.095 |
| XX | 0.008 | 0.036 | 0.014 | - | 0.12 |
| XXIII | 0.0009 | 0.0031 | 0.0021 | - | 0.027 |
| XXIV | 0.0071 | 0.017 | 0.011 | - | 0.034 |
| XXV | 0.0005 | 0.002 | 0.003 | - | 0.007 |

**Ansprüche**

1. Anthracyclin-Derivate, die der nachfolgenden allgemeinen Formel I

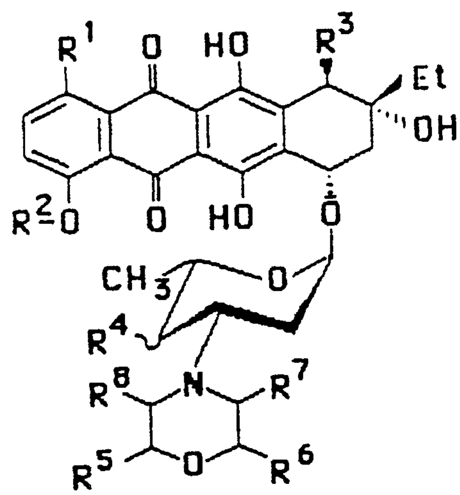

Formel I

entsprechen, in der die Reste folgende Bedeutung haben:
$R^1$ ist ein Wasserstoffatom oder eine Hydroxygruppe,
$R^2$ ist ein Wasserstoffatom oder eine Methylgruppe,
$R^3$ ist eine Methyloxycarbonyl- oder eine Hydroxygruppe,
$R^4$ ist ein Wasserstoffatom oder eine Hydroxygruppe,
$R^5$ ist ein Wasserstoffatom, eine Methylgruppe oder eine Hydroxymethylgruppe,
$R^6$ ist eine $C_{1-4}$-Alkyloxygruppe, Azidogruppe oder Cyanogruppe oder ein 9-N-Adenin-, 9-N-Guanin-, 1-N-Cytosin-, 1-N-Uracil- oder 1-N-Thymin-Rest,
$R^6$ kann auch ein Wasserstoffatom sein, wenn $R^1$ = OH und $R^3$ = $COOCH_3$ sind,
$R^5$ und $R^6$ sind zusammen $-O-CH_2-$ und
$R^7$ oder $R^8$ sind ein Wasserstoffatom oder eine Cyanogruppe.

2. Verbindung nach Anspruch 1, worin $R^6$ $OCH_3$, CN, $N_3$ oder 9-N-Adenin ist oder

$R^5$ und $R^6$ zusammen $-O-CH_2-$ sind und die übrigen Reste die in Anspruch 1 angegebene Bedeutung haben.

3. Anthracyclin-Derivat nach Anspruch 1 als Arzneimittel.

4. Verfahren zur Herstellung einer Verbindung nach Anspruch 1,

**dadurch gekennzeichnet,**

daß man ein Rhodomycinglycosid der Formel IV,

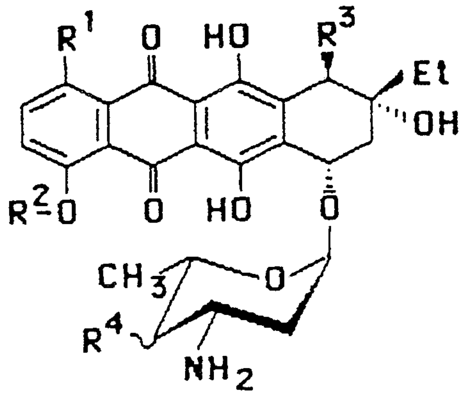

Formel IV

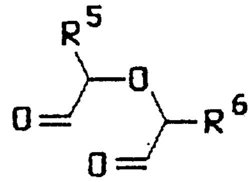

Formel III

worin

$R^1$ ein Wasserstoffatom oder eine Hydroxygruppe,

$R^2$ ein Wasserstoffatom oder eine Methylgruppe,

$R^3$ eine Methyloxycarbonyl- oder eine Hydroxygruppe und

$R^4$ ein Wasserstoffatom oder eine Hydroxygruppe sind,

oder eines seiner Additionssalze mit einem Überschuß eines Dialdehydes der Formel III, worin die Reste

$R^5$ ein Wasserstoffatom, eine Methylgruppe oder eine Hydroxymethylgruppe,

$R^6$ ein Wasserstoffatom, eine $C_{1-4}$-Alkyloxygruppe, Azidogruppe oder Cyano-gruppe oder einen 9-N-Adenin-, 9-N-Guanin-, 1-N-Cytosin-, 1-N-Uracil oder 1-N-Thymin-Rest oder

$R^5$ und $R^6$ zusammen $-O-CH_2-$ bedeuten,

in Anwesenheit von 1 bis 6 Äquivalenten eines Alkalimetallcyanoborhydrides und eines polaren organischen Lösungsmittels wie Methanol, Acetonitril oder dessen wäßrigen Gemischen bei 10° C bis 50° C zu einem Produktgemisch, bestehend aus einem Morpholino-Derivat der Formel I, worin die Reste $R^1$, $R^2$, $R^3$, $R^4$, $R^5$ und $R^6$ ihre vorher genannte Bedeutung beibehalten und $R^7$ und $R^8$ Wasserstoffatome sind, und einem Cyano-Morpholino-Derivat der Formel I, worin die Reste $R^1$, $R^2$, $R^3$, $R^4$, $R^5$ und $R^6$ ihre vorher genannte Bedeutung beibehalten und $R^7$ oder $R^8$ ein Wasserstoffatom oder eine Cyanogruppe ist, umsetzt, wobei die beiden Derivate isoliert werden.

Patentansprüche für folgende Vertragsstaaten: ES; GR

1. Verfahren zur Herstellung eines Anthracyclin-Derivats, der Formel I

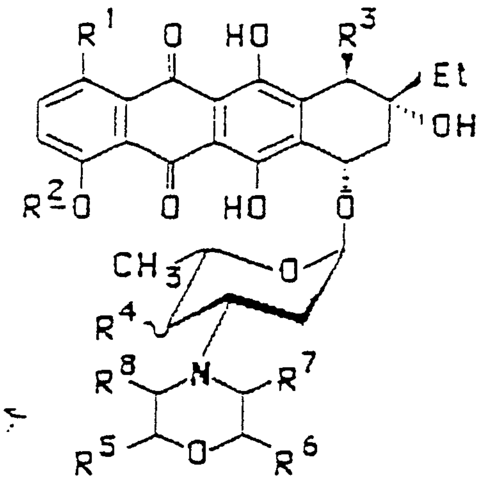

Formel I

in der die Reste folgende Bedeutung haben:
$R^1$ ist ein Wasserstoffatom oder eine Hydroxygruppe,
$R^2$ ist ein Wasserstoffatom oder eine Methylgruppe,
$R^3$ ist eine Methyloxycarbonyl- oder eine Hydroxygruppe,
$R^4$ ist ein Wasserstoffatom oder eine Hydroxygruppe,
$R^5$ ist ein Wasserstoffatom, eine Methylgruppe oder eine Hydroxymethylgruppe,
$R^6$ ist eine $C_{1-4}$-Alkyloxygruppe, Azidogruppe oder Cyanogruppe oder ein 9-N-Adenin-, 9-N-Guanin-, 1-N-Cytosin-, 1-N-Uracil- oder 1-N-Thymin-Rest,
$R^6$ kann auch ein Wasserstoffatom sein, wenn $R^1$ = OH und $R^3$ = $COOCH_3$ sind,
$R^5$ und $R^6$ sind zusammen $-O-CH_2-$ und
$R^7$ oder $R^8$ sind ein Wasserstoffatom oder eine Cyanogruppe,
**dadurch gekennzeichnet,**
daß man ein Rhodomycinglycosid der Formel IV,

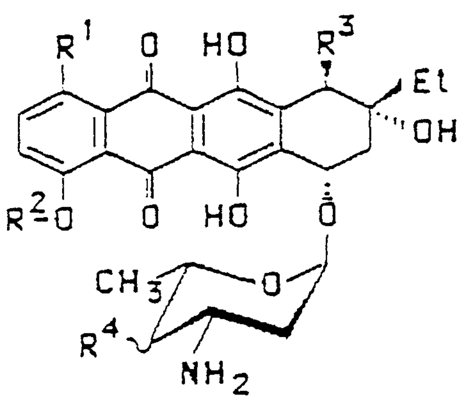

Formel IV

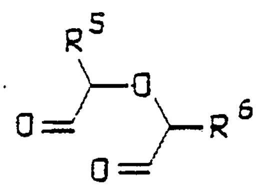

Formel III

worin
$R^1$ ein Wasserstoffatom oder eine Hydroxygruppe,
$R^2$ ein Wasserstoffatom oder eine Methylgruppe,
$R^3$ eine Methyloxycarbonyl- oder eine Hydroxygruppe und
$R^4$ ein Wasserstoffatom oder eine Hydroxygruppe sind,
oder eines seiner Additionssalze mit einem Überschuß eines Dialdehydes der Formel III, worin die Reste
$R^5$ ein Wasserstoffatom, eine Methylgruppe oder eine Hydroxymethylgruppe,
$R^6$ ein Wasserstoffatom, eine $C_{1-4}$-Alkyloxygruppe, Azidogruppe oder Cyano-gruppe oder einen 9-N-Adenin-, 9-N-Guanin-, 1-N-Cytosin-, 1-N-Uracil oder 1-N-Thymin-Rest oder
$R^5$ und $R^6$ zusammen $-O-CH_2-$ bedeuten,
in Anwesenheit von 1 bis 6 Äquivalenten eines Alkalimetallcyanoborhydrides und eines polaren

organischen-Lösungsmittels, vorzugsweise Methanol oder Acetonitril oder dessen wäßrigen Gemischen bei 10° C bis 50° C zu einem Produktgemisch, bestehend aus einem Morpholino-Derivat der Formel I, worin die Reste $R^1$, $R^2$, $R^3$, $R^4$, $R^5$ und $R^6$ ihre vorher genannte Bedeutung beibehalten und $R^7$ und $R^8$ Wasserstoffatome sind, und einem Cyano-Morpholino-Derivat der Formel I, worin die Reste $R^1$, $R^2$, $R^3$, $R^4$, $R^5$ und $R^6$ ihre vorher genannte Bedeutung beibehalten und $R^7$ oder $R^8$ ein Wasserstoffatom oder eine Cyanogruppe ist, umsetzt, und die beiden Derivate isoliert.